# EUROPEAN PATENT APPLICATION

(11) **EP 4 470 462 A1**
(43) Date of publication of application: **04.12.2024**
(21) Application number: 24177546.9
(22) Date of filing: 23.05.2024
(51) Int. Cl.: A61B 5/145, A61B 5/1473, A61B 5/1495, H04W 12/06, A61B 5/00, H04L 67/12, H04W 4/38, H04W 4/80, H04W 12/50

(54) **ANALYTE MONITORING DEVICE AND CONTROLLING METHOD THEREOF**

(30) Priority: 01.06.2023 KR 20230070642
(71) Applicant: I-SENS, INC., Seoul 06646 (KR)
(72) Inventor: LEE, Jin Won, 06646 Seoul (KR); LEE, Yunho, 06646 Seoul (KR)
(74) Representative: Westphal, Mussgnug & Partner Patentanwälte mbB

(57) **Abstract**

An analyte monitoring device is disclosed. The analyte monitoring device includes an analyte sensor, a communication interface, a memory, and at least one processor, in which the at least one processor activates a communication interface at a predetermined first time interval and performs authentication for a first signal when the first signal is received from the user terminal device, establishes a communication channel between the analyte monitoring device and the user terminal device when the authentication is successful, and transmits information related to an analyte signal to the user terminal device at a predetermined second time period through the communication channel.

## Description

### CROSS-REFERENCE TO RELATED APPLICATIONS

This application claims priority from Korean Patent Application No. 10-2023-0070642 filed in the Korean Intellectual Property Office on June 1, 2023, the disclosures of which are incorporated herein by reference.

### BACKGROUND

### 1. Technical Field

The present disclosure relates to an analyte monitoring device, and more particularly, to a continuous glucose monitoring (CGM) device and a controlling method thereof.

### 2. Related Art

Continuous glucose monitoring systems (CGMSs) are systems that acquire blood sugar concentrations of users using sensors in contact with body fluids (e.g., interstitial fluid) of the users and provide the acquired blood sugar concentrations to the users. A CGM system includes CGM device attached to the body of a user to sense signals from the body fluid of the user, and a user terminal device that provides the blood sugar concentration to the user.

The CGM device includes a sensor electronic unit including a battery and an analyte sensor inserted into the skin of a user. The existing CGM has a form in which the sensor electronic unit and the analyte sensor are structurally/electrically separated to prevent battery consumption before actual use. Here, the sensor electronic unit and the analyte sensor are combined while the user attaches the CGM device to the body, thereby setting up an electrical connection between the sensor electronic unit and the analyte sensor.

However, since this structure requires a structure to combine the sensor electronic unit and the analyte sensor, there are limitations in simplifying the structure of the CGM device and making the CGM device easier to manufacture.

Therefore, there is a need for a CGM device having a simpler structure while preventing excessive consumption of a battery before actual use.

### SUMMARY

The present disclosure provides a continuous glucose monitoring device in which excessive consumption of a battery is prevented before actual use and a controlling method thereof.

The present disclosure provides a continuous glucose monitoring device having a simple structure while preventing excessive consumption of a battery before actual use.

Objects of the present disclosure are not limited to the above-mentioned objects. That is, other objects that are not described may be obviously understood by those skilled in the art to which the present disclosure pertains from the following description.

According to an embodiment of the present disclosure, an analyte monitoring device includes: an analyte sensor of which at least a portion is located within a body of a user and detects an analyte signal related to a glucose concentration; a communication interface including at least one communication circuit; a memory storing at least one instruction; and at least one processor, in which the at least one processor executes the at least one instruction to activate the communication interface at a predetermined first time interval, perform authentication for a first signal when the first signal for waking up the analyte monitoring device is received from a user terminal device through the activated communication interface, establish a communication channel between the analyte monitoring device and the user terminal device when the authentication is successful, and transmit information related to the analyte signal to the user terminal device at a predetermined second time interval through the communication channel.

The first signal may include first identification information for establishing the communication channel.

The at least one processor may establish the communication channel based on whether the first identification information matches second identification information pre-stored in the memory.

The at least one processor may demodulate a first portion modulated based on on-off keying (OOK) in a packet of the first signal to acquire a first value, and determine that the authentication is successful when the first value matches a second value pre-stored in the memory.

The at least one processor may not perform signal processing on the analyte signal while activating the communication interface at the predetermined first time interval, and the signal processing may include at least one of analog-to-digital conversion, noise filtering, and calibration.

When a strength of the first signal is less than a preset value or the authentication fails, the at least one processor may activate the communication interface at the predetermined first time interval without establishing the communication channel.

The at least one processor may not perform the authentication when the strength of the first signal is less than the preset value.

The first time interval may be less than the second time interval.

The at least one processor may transmit calibration information used to correct the analyte signal to the user terminal device through the communication channel.

According to another embodiment of the present disclosure, a controlling method of an analyte monitoring device including an analyte sensor of which at least a portion is located within a body of a user and detects an analyte signal related to a glucose concentration includes: activating a communication interface included in the analyte monitoring device at a predetermined first time interval; performing authentication for a first signal when the first signal for waking up the analyte monitoring device is received from a user terminal device through the activated communication interface; establishing a communication channel between the analyte monitoring device and the user terminal device when the authentication is successful; and transmitting information related to the analyte signal to the user terminal device at a predetermined second time interval through the communication channel.

The first signal may include first identification information for establishing the communication channel, and the establishing of the communication channel may be performed based on whether the first identification information matches second identification information pre-stored in the memory.

The performing of the authentication may include: demodulating a first portion modulated based on on-off keying (OOK) in a packet of the first signal to acquire a first value; and determining that the authentication is successful when the first value matches a second value pre-stored in the memory.

The controlling method may further include when strength of the first signal is less than a preset value or the authentication fails, activating the communication interface at the predetermined first time interval without establishing the communication channel.

When the strength of the first signal is less than the preset value, the authentication may not be performed.

The controlling method may further include transmitting calibration information used to correct the analyte signal to the user terminal device through the communication channel.

Technical solutions of the present disclosure are not limited to the above-mentioned solutions, and solutions that are not mentioned will be clearly understood by those skilled in the art to which the present disclosure pertains from the present specification and the accompanying drawings.

### BRIEF DESCRIPTION OF DRAWINGS

Aspects, features, and advantages of specific embodiments of the present disclosure will become more apparent from the following description with reference to the accompanying drawings:
FIG. 1 is a schematic diagram illustrating a continuous glucose monitoring (CGM) system according to an embodiment of the present disclosure;
FIG. 2A is a schematic diagram illustrating an analyte monitoring device according to a first embodiment that is inserted into the skin of a user;
FIG. 2B is a schematic diagram illustrating an analyte monitoring device according to a second embodiment that is inserted into the skin of a user;
FIG. 2C is a schematic diagram illustrating an analyte monitoring device according to a third embodiment that is inserted into the skin of a user;
FIG. 3 is a flowchart of a controlling method of an analyte monitoring device according to an embodiment of the present disclosure;
FIG. 4 is a sequence diagram illustrating an operation of an analyte monitoring system according to an embodiment of the present disclosure;
FIG. 5 is a diagram for describing a controlling method of an electronic device according to an embodiment of the present disclosure; and
FIG. 6 is a block diagram illustrating a configuration of an analyte monitoring system according to an embodiment of the present disclosure.

### DESCRIPTION OF EXAMPLE EMBODIMENTS

After terms used in the present specification are briefly described, the present disclosure will be described in detail.

General terms that are currently widely used were selected as terms used in embodiments of the present disclosure in consideration of functions in the present disclosure, but may be changed according to the intention of those skilled in the art or a judicial precedent, the emergence of a new technique, and the like. In addition, in a specific case, terms arbitrarily chosen by an applicant may exist. In this case, the meaning of such terms will be mentioned in detail in a corresponding description portion of the present disclosure. Therefore, the terms used in the present disclosure should be defined on the basis of the meaning of the terms and the contents throughout the present disclosure rather than simple names of the terms.

The present disclosure may be variously modified and have several embodiments, and therefore specific embodiments of the present disclosure will be illustrated in the drawings and be described in detail in the detailed description. However, it is to be understood that the present disclosure is not limited to specific exemplary embodiments, but includes all modifications, equivalents, and substitutions without departing from the scope and spirit of the present disclosure. When it is determined that a detailed description of the known art related to the present disclosure may obscure the gist of the present disclosure, the detailed description will be omitted.

The terms "first," "second," and the like may be used to describe various components, but the components are not to be construed as being limited by these terms. The terms are used only to distinguish one component from another component.

Singular forms are intended to include plural forms unless the context clearly indicates otherwise. It should be understood that terms "include" or "comprise" used in the present specification, specify the presence of features, numerals, steps, operations, components, parts mentioned in the present specification, or combinations thereof, but do not preclude the presence or addition of one or more other features, numerals, steps, operations, components, parts, or combinations thereof.

Hereinafter, embodiments of the present disclosure will be described in detail with reference to the accompanying drawings so that those skilled in the art to which the present disclosure pertains may easily practice the present disclosure. However, the present disclosure may be modified in various different forms, and is not limited to embodiments described herein. In addition, in the drawings, portions unrelated to the description will be omitted to obviously describe the present disclosure, and similar reference numerals will be used to describe similar portions throughout the specification.

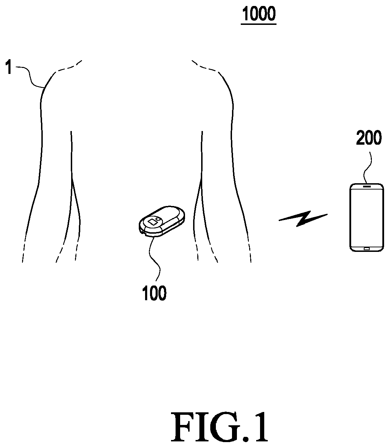

FIG. 1 is a schematic diagram illustrating a continuous glucose monitoring (CGM) system according to an embodiment of the present disclosure.

Referring to FIG. 1, an analyte monitoring system 1000 may include an analyte monitoring device 100 and a user terminal device 200. For example, the analyte monitoring system 1000 may be a CGM system (CGMS), and the analyte monitoring device 100 may be a CGM device. The user terminal device 200 may be a smart phone, a tablet PC, a smart watch, personal digital assistant (PDA), or a dedicated receiver (e.g., receiver).

The analyte monitoring device 100 may acquire information related to a concentration of an analyte included in a body fluid of a user 1. The analyte may include glucose and ketone. Information related to the concentration of the analyte may include a size of a signal related to the concentration of the analyte and a value representing the concentration.

The analyte monitoring device 100 may be attached to the body of the user 1. At least a portion of the analyte monitoring device 100 may be inserted into a skin of the user 1 and located within a body of the user 1.

The user terminal device 200 may receive the information related to the concentration of the analyte from the analyte monitoring device 100. The user terminal device 200 may provide the information related to the concentration of the analyte to the user. The user terminal device 200 may provide the information related to the concentration of the analyte to a display. The information related to the concentration of the analyte provided to the user may include data processed as a signal by the user terminal device 200. For example, the user terminal device 200 may apply a predefined algorithm to the signal sensed by the analyte monitoring device 100 to generate a blood sugar concentration value.

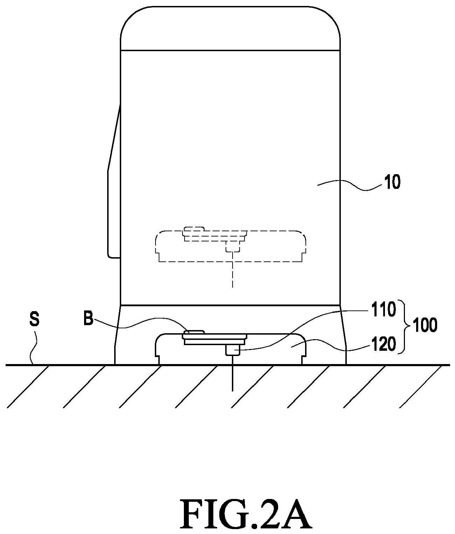

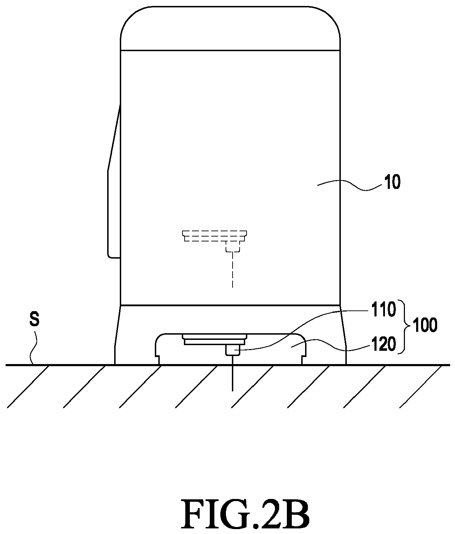

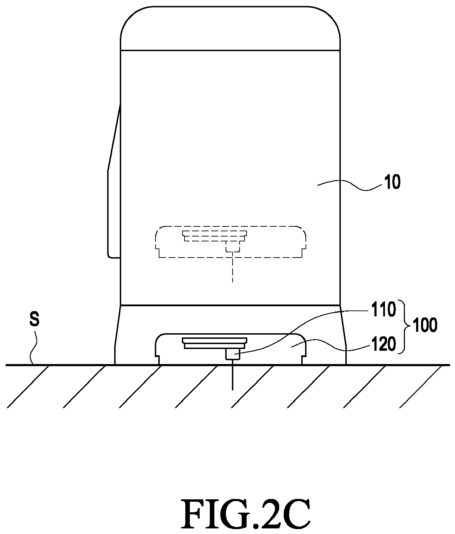

FIG. 2A is a schematic diagram illustrating an analyte monitoring device according to a first embodiment that is inserted into the skin of a user. FIG. 2B is a schematic diagram illustrating an analyte monitoring device according to a second embodiment that is inserted into the skin of a user. FIG. 2C is a schematic diagram illustrating an analyte monitoring device according to a third embodiment that is inserted into the skin of a user.

Referring to FIGS. 2A to 2C, the analyte monitoring device 100 may include an analyte sensor 110 and a sensor electronic unit 120. The analyte sensor 110 and/or the sensor electronic unit 120 may be moved by an applicator 10. The analyte sensor 110 may be inserted into the skin S of a user by the applicator 10.

In the first and third embodiments, the applicator 10 may move the analyte sensor 110 and the sensor electronic unit 120. In the second embodiment, the applicator 10 may move the analyte sensor 110. For example, in the first embodiment, the analyte monitoring device 100 may be located at a first position spaced apart from the skin S by a predetermined distance inside the applicator 10. When the applicator 10 operates by the user, the applicator 10 may move the analyte monitoring device 100 to a second position.

The applicator 10 may be operated by the user while one open side of the applicator 10 is in close contact with the skin S. The applicator 10 may include a needle (not illustrated) formed to surround one end of the analyte sensor 110 inside, a first elastic member (not illustrated) that pushes the needle and one end of the analyte sensor 110 together to the skin S, and a second elastic member (not illustrated) for extracting only the needle. The needle and one end of the analyte sensor 110 may be simultaneously inserted into the skin S by decompressing the first elastic member (not illustrated) that is disposed in a compressed state inside the applicator 10. When one end of the analyte sensor 110 is inserted into the skin S, the needle may be pulled out by decompressing the compressed second elastic member (not illustrated), and the analyte sensor 110 may remain inserted into the skin S.

An adhesive tape may be provided on a body contact surface of the sensor electronic unit 120 so that the sensor electronic unit 120 may be fixedly attached to the skin S of the body. Accordingly, when the applicator 10 is spaced apart from the skin S of the body, the sensor electronic unit 120 may be fixedly attached to the skin S of the body by the adhesive tape.

In the first embodiment, the analyte monitoring device 100 may include a button B for electrically connecting the analyte sensor 110 and the sensor electronic unit 120. Before the user presses the button B, the sensor electronic unit 120 may be in an open circuit state. In addition, the analyte sensor 110 may be spaced apart from the sensor electronic unit 120 and not electrically connected to the sensor electronic unit 120. When the user presses the button B, the analyte sensor 110 may be electrically connected to the sensor electronic unit 120 to form a closed circuit. Accordingly, the battery included in the sensor electronic unit 120 may supply power to other components (e.g., a communication interface) of the sensor electronic unit 120.

In the second embodiment, the analyte monitoring device 100 may not include the button B. In this case, the analyte sensor 110 may be coupled to the sensor electronic unit 120 in an open circuit state by the applicator 10. The closed circuit may be formed by coupling the analyte sensor 110 and the sensor electronic unit 120. Accordingly, the battery included in the sensor electronic unit 120 may supply power to other components of the sensor electronic unit 120.

In the third embodiment, the analyte sensor 110 and the sensor electronic unit 120 may be coupled from an initial state before the applicator 10 operates and electrically connected. The sensor electronic unit 120 may perform operations for communication connection with the user terminal device 200 while operating in a sleep mode using minimal power.

The analyte monitoring device 100 according to the third embodiment may have a simpler structure than the first and second embodiments. Since there is no need to move the analyte sensor 110 and couple/connect the analyte sensor 110 to the sensor electronic unit 120 during use by the user 1, for example, the analyte sensor 110 and the sensor electronic unit 120 may be manufactured integrally. Accordingly, the third embodiment may have lower manufacturing difficulty or cost compared to the first and second embodiments.

In the case of the analyte monitoring device 100 according to the third embodiment, since the battery is being consumed before the user 1 uses the analyte monitoring device 100, there is a need for a controlling method of preventing excessive consumption of the battery. For example, the sleep mode may be applied to the analyte monitoring device 100. The sleep mode (or low power mode) refers to a mode that operates using only minimal power. In the sleep mode, some operations that the analyte monitoring device 100 performs in a connecting mode may not be performed. For example, the analyte monitoring device 100 may not perform signal processing on the analyte signal acquired using the analyte sensor 110 in the sleep mode. In addition, in order to reduce power consumption, the analyte monitoring device 100 may intermittently activate a communication interface in the sleep mode to wait for transmission of a wake-up signal from the user terminal device 200.

Hereinafter, the operation and controlling method of the analyte monitoring device 100 related to the sleep mode will be described. Meanwhile, although the sleep mode is applied to the analyte monitoring device 100 according to the third embodiment as an example, it should be noted that the embodiment to which the sleep mode according to the present disclosure is applied is not limited to the third embodiment. In other words, the sleep mode may be applied to the analyte monitoring device 100 according to the first or second embodiments and contribute to reducing the battery consumption.

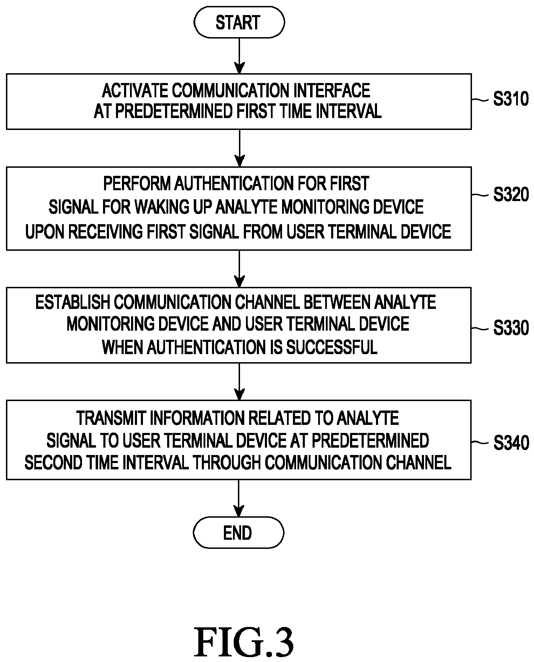

FIG. 3 is a flowchart of a controlling method of an analyte monitoring device according to an embodiment of the present disclosure.

Referring to FIG. 3, the analyte monitoring device 100 may activate the communication interface at a predetermined first time interval (S310). In this case, the analyte monitoring device 100 may operate in a first mode. The first mode may be the sleep mode (or low power mode). The analyte monitoring device 100 may wait for reception of a radio frequency (RF) signal in a specific frequency band (e.g., 2.4 GHz). The communication interface may include an RF receiver for receiving the RF signal. Meanwhile, the first time interval may be preset to a value that may prevent the excessive battery consumption of the analyte monitoring device 100 while enabling the smooth communication connection between the analyte monitoring device 100 and the user terminal device 200.

When the first signal is received from the user terminal device, the analyte monitoring device 100 may perform authentication for the first signal (S320). The first signal may be the wake-up signal. The wake-up signal may be modulated according to an on-off keying (OOK) method. A packet (or wake-up packet) of the wake-up signal may include a first portion modulated based on the OOK. The analyte monitoring device 100 may acquire a first value by demodulating the first portion. The analyte monitoring device 100 may determine whether the first value matches a second value pre-stored in the memory. The first value may be generated using an application (e.g., a dedicated app for a CGM) corresponding to the analyte monitoring device 100 installed in the user terminal device 200. The second value may be a value preset by a manufacturer and stored in the memory of the analyte monitoring device 100.

When the first value and the second value match, the analyte monitoring device 100 may determine that the authentication for the wake-up signal succeeds. When the first value and the second value mismatch, the analyte monitoring device 100 may determine that the authentication for the wake-up signal fails. That is, the analyte monitoring device 100 may be woken up only through the app provided for the analyte monitoring device 100. Accordingly, it is possible to prevent the analyte monitoring device 100 from being woken up by any device unrelated to the analyte monitoring device 100.

The analyte monitoring device 100 may determine whether the strength of the wake-up signal is greater than or equal to a preset value. When the strength of the wake-up signal is less than the preset value, the analyte monitoring device 100 may not perform the authentication for the wake-up signal. Alternatively, when the strength of the wake-up signal is less than the preset value, it may be determined that the authentication for the wake-up signal fails.

The wake-up packet may include a second portion including identification information for communication connection between the analyte monitoring device 100 and an external device (e.g., user terminal device 200). The identification information is information that may be used for mutual authentication between the analyte monitoring device 100 and the external device, and may include, for example, a passkey.

When the authentication for the first signal is successful, the analyte monitoring device 100 may establish a communication channel between the analyte monitoring device 100 and the user terminal device 200 (S330). For example, the analyte monitoring device 100 may perform the communication connection according to a Bluetooth low energy (BLE) protocol. Specifically, the analyte monitoring device 100 may enter the second mode to transmit an advertising packet to a peripheral device. The second mode may be an advertising mode.

The advertising mode may refer to a mode in which the analyte monitoring device 100 periodically transmits the advertising signal to the outside for communication connection with the external device such as the user terminal device 200. In the advertising mode, the analyte monitoring device 100 may perform broadcasting to identify the user terminal device 200. The analyte monitoring device 100 may perform pairing based on the identification information received from the user terminal device 200.

The analyte monitoring device 100 may transmit information related to the analyte signal to the user terminal device 200 at a predetermined second time interval (S340). When the analyte monitoring device 100 is paired with the user terminal device 200, the analyte monitoring device 100 may enter a third mode. The third mode may be a connecting mode. In the connecting mode, the analyte monitoring device 100 may transmit the information related to the analyte signal to the user terminal device 200 at a second time interval. The user terminal device 200 may provide the information related to the analyte signal received at every second time interval to the user.

Meanwhile, the second time interval may be preset to a value that may provide the information related to the analyte signal to the user in substantially real time. In an embodiment, the second time interval may be greater than the first time interval. For example, the first time interval may be 1 minute and the second time interval may be 5 minutes. In other embodiments, the first time interval and the second time interval may be equal, or the first time interval may be greater than the second time interval.

Meanwhile, the first time interval and/or the second time interval may be variable. For example, the first time interval and/or the second time interval may vary depending on the remaining amount of a battery 124. Specifically, as the remaining amount of the battery 124 decreases, the first time interval and/or the second time interval may increase to reduce the usage of the battery 124. As another example, the first time interval and/or the second time interval may vary depending on a difference between the manufacturing time of the analyte monitoring device 100 and the current time. Specifically, as the difference between the manufacturing time and the current time increases, the first time interval and/or the second time interval may increase. The analyte monitoring device 100 may measure the remaining amount of the battery 124. The analyte monitoring device 100 may measure the current time and difference between the manufacturing time of the analyte monitoring device 100, and the current time.

The information related to the analyte signal may include the size of the analyte signal or the concentration value of the analyte. In the connecting mode, the analyte monitoring device 100 may perform signal processing on the analyte signal. For example, the analyte monitoring device 100 may convert an analyte signal into a digital signal. The analyte monitoring device 100 may perform noise filtering on the analyte signal. In addition, the analyte monitoring device 100 may perform calibration on the analyte signal to calculate the concentration value of the analyte.

In the present disclosure, the first mode, the second mode, and the third mode are used as examples of the operation mode of the analyte monitoring device 100, but various other operation modes may exist. For example, a fourth mode may be an operation mode in which the analyte monitoring device 100 is not paired with the user terminal device 200, but is performing various signal processing. In this case, the analyte monitoring device 100 may sense, process, and store the analyte signal in real time. Thereafter, when the operation mode of the analyte monitoring device 100 is in the third mode, the data stored in the meantime may be transmitted to the user terminal device 200.

The entry from the third mode to the fourth mode may occur periodically. For example, the analyte monitoring device 100 may transmit the information related to the analyte signal to the user terminal device 200 at every predetermined interval and then enter the fourth mode. Then, the analyte monitoring device 100 may enter the second mode according to a predetermined schedule to perform the advertising.

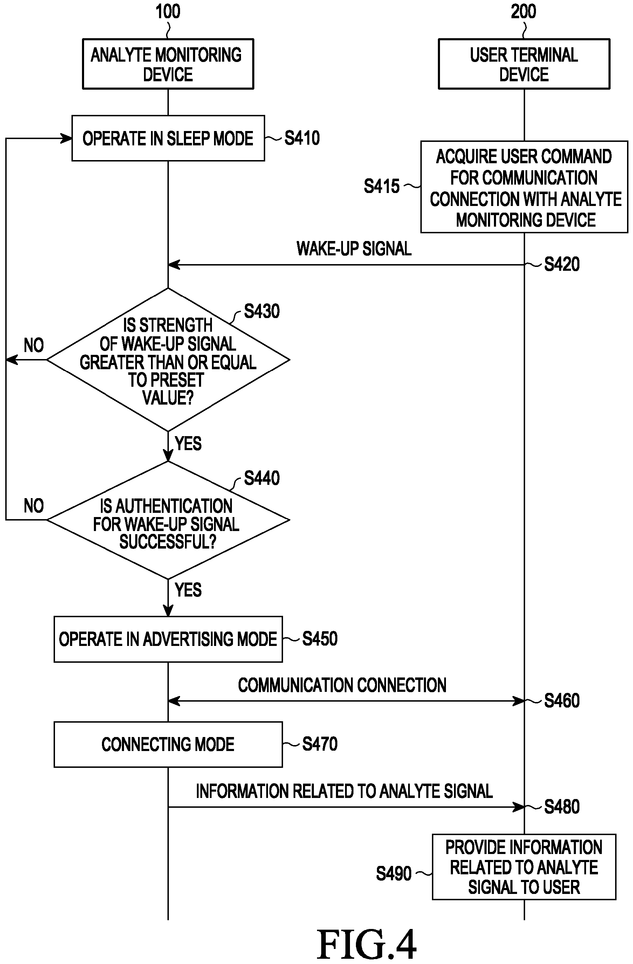

FIG. 4 is a sequence diagram illustrating an operation of an analyte monitoring system according to an embodiment of the present disclosure.

Referring to FIG. 4, the analyte monitoring device 100 may operate in the sleep mode (S410). In this case, the analyte monitoring device 100 may wait for the reception of the wake-up signal while activating the communication interface at a predetermined time period.

While the analyte monitoring device 100 operates in the sleep mode, the user terminal device 200 may acquire a user command for communication connection with the analyte monitoring device 100 (S415). When the user command is acquired, the user terminal device 200 may generate the wake-up signal. In this case, the user terminal device 200 may modulate at least part of the wake-up signal using the OOK method. The user terminal device 200 may transmit the wake-up signal to the analyte monitoring device 100 (S420).

The analyte monitoring device 100 may determine whether the strength of the wake-up signal is greater than or equal to a preset value (S430). When the strength of the wake-up signal is greater than or equal to the preset value (S430-Y), the analyte monitoring device 100 may not perform the authentication for the wake-up signal (S440). When the strength of the wake-up signal is less than the preset value (S430-N), the analyte monitoring device 100 may maintain the sleep mode without performing the authentication for the wake-up signal.

When the authentication for the wake-up signal succeeds (S440-Y), the analyte monitoring device 100 may operate in the advertising mode (S450). In this case, the analyte monitoring device 100 may perform the communication connection with the user terminal device 200 (S460). Specifically, the analyte monitoring device 100 may perform the broadcasting to identify the user terminal device 200. The analyte monitoring device 100 may perform a pairing process with the user terminal device 200. The pairing process may be performed according to the BLE protocol.

The identification information (e.g., passkey) used in the pairing process may be included in the wake-up signal. Alternatively, the user terminal device 200 may transmit the identification information to the analyte monitoring device 100 in the communication connection process (S460). The identification information may be acquired in various ways. As an example, the identification information may be a QR code on wrapping paper of the analyte monitoring device 100. In this case, the user may scan the QR code using a camera of the user terminal device 200, so the user terminal device 200 may acquire the identification information. As another example, it is possible for the user to input the identification information to the user input unit of the user terminal device 200. As another example, the user terminal device 200 may receive the identification information from the external server.

When the communication connection between the analyte monitoring device 100 and the user terminal device 200 is completed, the analyte monitoring device 100 may enter the connecting mode (S470). In the connecting mode, the analyte monitoring device 100 may transmit the information related to the analyte signal to the user terminal device 200 (S480). In the connecting mode, the analyte monitoring device 100 may perform signal processing on the analyte signal acquired through the analyte sensor. The analyte monitoring device 100 may transmit the signal-processed analyte signal to the user terminal device 200.

The user terminal device 200 may provide the information related to the analyte signal to the user (S490). The user terminal device 200 may display a blood sugar value of a user on the display. In addition, the user terminal device 200 may perform additional processing on the information related to the analyte signal received from the analyte monitoring device 100. For example, when the information related to the analyte signal received from the analyte monitoring device 100 is a voltage value representing the blood sugar value of the user, the user terminal device 200 may apply the voltage value to a predefined algorithm to calculate the blood sugar value. The user terminal device 200 may display the calculated blood sugar value.

Meanwhile, in FIG. 4, it is illustrated that the analyte monitoring device 100 is woken up according to the command of S415 to perform the communication connection operation (S460). In this way, a command to wake-up the analyte monitoring device 100 and a command for communication connection may be formed as one. According to another embodiment, a command for waking up the analyte monitoring device 100 and a command for communication connection may exist separately. For example, prior to S460, the user terminal device 200 may receive a user command for communication connection from the user.

In the connecting mode, the analyte monitoring device 100 may transmit the calibration information to the user terminal device 200. The user terminal device 200 may calculate the blood sugar value of the user from the analyte signal based on the calibration information. The calibration information may include parameter values for compensating for time delay due to diffusion of glucose between an interstitial fluid and blood of a user. The parameter values may be calculated through in-vivo testing and stored in the analyte monitoring device 100.

The calibration operation for the analyte signal may be performed by the analyte monitoring device 100. For example, in the connecting mode, the analyte monitoring device 100 may receive reference data from the user terminal device 200. The reference data may be the blood sugar value of the user measured through a blood glucose meter. The analyte monitoring device 100 may calculate the blood sugar value of the user from the analyte signal using the blood sugar value received from the user terminal device 200 and the pre-stored parameter values.

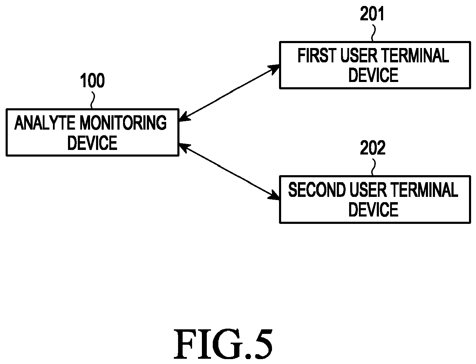

FIG. 5 is a diagram for describing a controlling method of an electronic device according to an embodiment of the present disclosure.

Referring to FIG. 5, the analyte monitoring device 100 may communicate with a plurality of user terminal devices 201 and 202. For example, the first user terminal device 201 may be a receiver that provides blood sugar information received from the analyte monitoring device 100 to the user, and the second user terminal device 202 may be a smartphone. However, this is an example, and the first user terminal device 201 and the second user terminal device 202 may be the same type of devices.

Each of the first user terminal device 201 and the second user terminal device 202 may have authority to wake-up the analyte monitoring device 100 (also referred to as wake-up authority for the analyte monitoring device 100). That is, each of the first user terminal device 201 and the second user terminal device 202 may generate the wake-up signal and transmit the generated wake-up signal to the analyte monitoring device 100. For example, the first user terminal device 201 may generate a first wake-up signal. The second user terminal device 202 may generate a second wake-up signal.

The analyte monitoring device 100 may wake-up based on the wake-up signal received first among the wake-up signals transmitted by the user terminal devices 201 and 202, respectively. For example, when the first wake-up signal is received before the second wake-up signal and the authentication for the first wake-up signal is completed, the analyte monitoring device 100 may wake-up based on the first wake-up signal to perform the communication connection with the user terminal device 201. When the operation mode of the analyte monitoring device 100 is not in the sleep mode, the analyte monitoring device 100 may receive a second wake-up signal. In this case, the analyte monitoring device 100 may ignore the second wake-up signal.

A device having the wake-up authority for the analyte monitoring device 100 may be predetermined. For example, the receiver may not have the wake-up authority, but the smartphone may have the wake-up authority. Upon receiving the wake-up signal, the analyte monitoring device 100 may determine whether the device transmitting the wake-up signal has the wake-up authority.

The analyte monitoring device 100 may determine whether the device transmitting the wake-up signal has the wake-up authority based on the identification information (e.g., model number) or type information (e.g., whether the device is a receiver or a smartphone) of the device included in the wake-up signal packet. The analyte monitoring device 100 may compare the identification information or type information of the device included in the packet of the wake-up signal with the information pre-stored in the analyte monitoring device 100, and determine whether the device transmitting the wake-up signal has the wake-up authority depending on whether the identification information or type information matches the pre-stored information according to the comparison result. The pre-stored information is information on a predetermined device that has the wake-up authority for the analyte monitoring device 100, and may include the identification information or type information of the device.

For example, when the identification information included in the packet of the wake-up signal matches the pre-stored identification information, the analyte monitoring device 100 may determine that the device transmitting the wake-up signal has the wake-up authority. On the other hand, when the identification information included in the packet of the wake-up signal mismatches the pre-stored identification information, the analyte monitoring device 100 may determine that the device transmitting the wake-up signal does not have the wake-up authority.

It may be determined whether the device transmitting the wake-up signal to the analyte monitoring device 100 has the wake-up authority for the analyte monitoring device 100 based on app information included in the packet of the wake-up signal. The wake-up authority for the analyte monitoring device 100 may be granted only to the predetermined app. For example, the first app may have the wake-up authority, and the second app, which is a follower app for the first app, may not have the wake-up authority. When the wake-up signal is received, the analyte monitoring device 100 may determine whether the app information stored in the packet of the wake-up signal matches the pre-stored app information. When the app information matches the pre-stored app information, the analyte monitoring device 100 may determine that the device transmitting the wake-up signal has the wake-up authority. When the app information mismatches the pre-stored app information, the analyte monitoring device 100 may determine that the device transmitting the wake-up signal does not have the wake-up authority.

Meanwhile, a method for acquiring, by the first user terminal device 201 and the second user terminal device 202, authentication information for communication connection with the analyte monitoring device 100 may be different. For example, the authentication information acquired by the first user terminal device 201 may be acquired by the user directly inputting the authentication information to the input unit included in the first user terminal device 201. The authentication information acquired by the second user terminal device 202 may be acquired by scanning, by a camera, the QR code printed on the analyte monitoring device 100 or the wrapping paper of the analyte monitoring device 100.

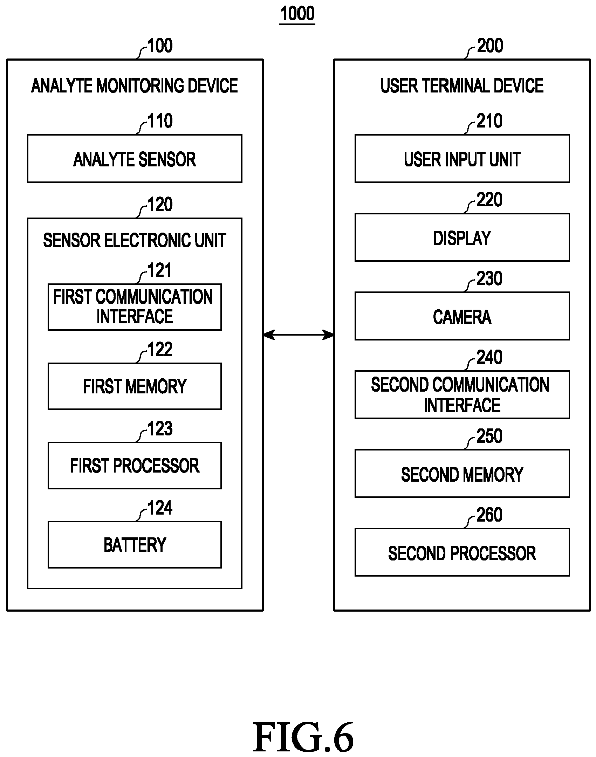

FIG. 6 is a block diagram illustrating a configuration of an analyte monitoring system according to an embodiment of the present disclosure.

Referring to FIG. 6, the analyte monitoring system 1000 may include the analyte monitoring device 100 and the user terminal device 200.

The analyte monitoring device 100 may include the analyte sensor 110 and the sensor electronic unit 120. The sensor electronic unit 120 may include a first communication interface 121, a first memory 122, a first processor 123, and a battery 124. For example, the analyte monitoring device 100 may be a CGM device.

The analyte sensor 110 may be configured to sense an analyte signal. The analyte sensor 110 may include at least a portion of a sensor probe inserted into a body. A sensing area that reacts with sugar within the body may be formed in the sensor probe to measure the blood sugar in the body.

The first communication interface 121 includes at least one communication circuit and may communicate with various types of external devices. For example, the first communication interface 121 may receive the wake-up signal from the user terminal device 200. In addition, the first communication interface 121 may transmit the information related to the analyte signal to the user terminal device 200. The first communication interface 121 may include a Bluetooth module, a low-power Bluetooth module, an RF module, and a near field communication (NFC) module.

The first memory 122 may store an operating system (OS) for controlling an overall operation of the components of the analyte monitoring device 100 and commands or data related to the components of the analyte monitoring device 100. In particular, the first memory 122 may store instructions for setting up the communication connection with the user terminal device 200. The first memory 122 may be implemented as a non-volatile memory (e.g., a hard disk, a solid state drive (SSD), and a flash memory), a volatile memory, or the like.

The first processor 123 may be electrically connected to the first memory 122 to control the overall function and operation of the analyte monitoring device 100. The first processor 123 may execute the instructions stored in the first memory 122 to control the analyte monitoring device 100.

The first processor 123 may operate in the first mode. The first mode may be the sleep mode (or low power mode). In this case, the first processor 123 may activate the first communication interface 121 at the first time interval to wait for the reception of the wake-up signal. In the first mode, the first processor 123 may not perform the signal processing on the analyte signal. Accordingly, the usage of the battery 124 may be saved.

When the wake-up signal is received through the first communication interface 121, the first processor 123 may perform the authentication for the wake-up signal. The first processor 123 may demodulate the first portion modulated based on the OOK in the packet of the wake-up signal to acquire the first value. When the first value matches the second value pre-stored in the first memory 122, the first processor 123 may determine that the authentication for the wake-up signal is successful.

When the authentication of the wake-up signal is successful, the first processor 123 may enter the advertising mode. The first processor 123 may perform the communication connection with the user terminal device 200 through the first communication interface 121. The first processor 123 may perform broadcasting using the first communication interface 121 to identify the user terminal device 200.

When the user terminal device 200 is identified, the first processor 123 may perform pairing with the user terminal device 200. The first processor 123 may exchange the authentication information for pairing with the user terminal device 200 through the first communication interface 121. For the authentication information for pairing, the analyte monitoring device 100 may include the identification information related to the user terminal device 200. The identification information may be included in the wake-up signal.

For example, the identification information may include first identification information. The first processor 123 may perform the pairing based on whether the first identification information matches the second identification information pre-stored in the first memory 122. When the first identification information matches (or is the same as) the second identification information, the first processor 123 may establish the communication channel between the analyte monitoring device 100 and the user terminal device 200. When the first identification information does not match (or is different from) the second identification information, the first processor 123 may not establish the communication channel between the analyte monitoring device 100 and the user terminal device 200.

When the communication channel is established, the first processor 123 may control the first communication interface 121 to transmit the information related to the analyte signal to the user terminal device 200 at the second time interval.

The analyte monitoring device 100 may include a plurality of processors. For example, the first communication interface 121 may be implemented as a BLE chip. In this case, the BLE chip may include a CPU different from the first processor 123. In the present disclosure, the operation mode of the first processor 123 may refer to the operation mode of the analyte monitoring device 100.

The battery 124 may provide power to the components of the analyte monitoring device 100. The consumption of the battery 124 may vary according to the operation mode of the analyte monitoring device 100. When the analyte monitoring device 100 is in the sleep mode, the consumption of the battery 124 may be less than the consumption of the battery 124 when the analyte monitoring device 100 is in the advertising mode. When the analyte monitoring device 100 is in the advertising mode, the consumption of the battery 124 may be less than the consumption of the battery 124 when the analyte monitoring device 100 is in the connecting mode.

The user terminal device 200 may include a user input unit 210, a display 220, a camera 230, a second communication interface 240, a second memory 250, and a second processor 260. For example, the user terminal device 200 may be a smartphone.

The user input unit 210 is configured to receive the user command. The user input unit 210 may receive the user command to wake-up the analyte monitoring device 100. The user input unit 210 may receive the user command for communication connection with the analyte monitoring device 100. The user command for waking up the analyte monitoring device 100 and the user command for communication connection with the analyte monitoring device 100 do not necessarily need to be separate commands. For example, according to the user command for communication connection with the analyte monitoring device 100, the analyte monitoring device 100 may be woken-up, and the communication connection between the analyte monitoring device 100 and the user terminal device 200 may be established.

The user input unit 210 may include a key pad, a dome switch, a touch pad (static pressure/electrostatic), a jog wheel, a jog switch, and a sensor (e.g., a voice sensor, a proximity sensor, an illuminance sensor, an acceleration sensor, a gyro sensor, etc.). The user input unit 210 may be implemented in the form of a button on the outside of the user terminal device 200, and some buttons may be implemented as a touch panel. When the user input (e.g., text input, voice input, change in user device operation, etc.) for rule definition and rule execution (e.g., command) is received, the user input unit 210 may generate the input data accordingly.

The display 220 may output the information related to the analyte signal. For example, the display 220 may output the blood sugar value of the user. In addition, the display 220 may output UI elements for receiving user commands. For example, the display 220 may output a user interface (UI) element for receiving the user command for communication connection with the analyte monitoring device 100.

The camera 230 may be used to acquire the identification information for authentication between the analyte monitoring device 100 and the user terminal device 200. For example, the user terminal device 200 may scan the QR code on the wrapping paper of the analyte monitoring device 100 using the camera 230.

The second communication interface 240 may include at least one communication circuit. The second communication interface 240 may transmit the wake-up signal to the first communication interface 121. The second communication interface 240 may receive the information related to the analyte signal from the first communication interface 121. The second communication interface 240 may include the Bluetooth module, the low-power Bluetooth module, the RF module, and the NFC module.

The second memory 250 may store the operating system (OS) for controlling the overall operation of the components of the user terminal device 200 and the commands or data related to the components of the user terminal device 200. In particular, the second memory 250 may store the instructions for setting up the communication connection with the analyte monitoring device 100. The second memory 250 may be implemented as the non-volatile memory (e.g., a hard disk, an SSD, and a flash memory), the volatile memory, or the like.

The second processor 260 may be electrically connected to the second memory 250 to control the overall function and operation of the user terminal device 200. The second processor 260 may control the user terminal device 200 by executing the instructions stored in the second memory 250.

The second processor 260 may acquire the user command for communication connection with the analyte monitoring device 100 through the user input unit 210. When the user command is input, the second processor 260 may generate a wake-up signal 420. The second processor 260 may modulate at least a portion of the wake-up signal 420 using the OOK method. The second processor 260 may control the second communication interface 240 to transmit the modulated wake-up signal 420 to the analyte monitoring device 100.

The second processor 260 may control the display 220 to display the information related to the analyte signal.

Various exemplary embodiments of the present disclosure described above may be implemented in a computer or a computer readable recording medium using software, hardware, or a combination of software and hardware. In some cases, embodiments described in the present disclosure may be implemented by the processor itself. According to a software implementation, embodiments such as procedures and functions described in the present disclosure may be implemented by separate software modules. Each of the software modules may perform one or more functions and operations described in the present disclosure.

Computer instructions for performing processing operations according to the diverse embodiments of the present disclosure described above may be stored in a non-transitory computer-readable medium. The computer instructions stored in the non-transitory computer-readable medium allow a specific machine to perform the processing operations according to the diverse embodiments described above when they are executed by a processor.

The non-transitory computer-readable medium is not a medium that stores data for a while, such as a register, a cache, a memory, or the like, but means a medium that semi-permanently stores data and is readable by the apparatus. A specific example of the non-transitory computer-readable medium may include a compact disk (CD), a digital versatile disk (DVD), a hard disk, a Blu-ray disk, a universal serial bus (USB), a memory card, a read only memory (ROM), or the like.

The machine-readable storage medium may be provided in the form of a non-transitory storage medium. Here, the "non-transitory storage medium" means that the storage medium is a tangible device, and does not include a signal (for example, electromagnetic waves), and the term does not distinguish between the case where data is stored semi-permanently on a storage medium and the case where data is temporarily stored thereon. For example, the "non-transitory storage medium" may include a buffer in which data is temporarily stored.

The methods according to the diverse embodiments disclosed in the document may be included and provided in a computer program product. The computer program product may be traded as a product between a seller and a purchaser. The computer program product may be distributed in the form of a machine-readable storage medium (for example, compact disc read only memory (CD-ROM)), or may be distributed (for example, download or upload) through an application store (for example, Play Store^{™}) or may be directly distributed (for example, download or upload) between two user devices (for example, smart phones) online. In a case of the online distribution, at least some of the computer program products (for example, downloadable app) may be at least temporarily stored in a machine-readable storage medium such as a memory of a server of a manufacturer, a server of an application store, or a relay server or be temporarily created.

According to various embodiments of the present disclosure as described above, a CGM device can have a simple structure while preventing excessive consumption of the battery before actual use. Accordingly, it is possible to reduce difficulty and cost of manufacturing the CGM device.

In addition, effects obtainable or predicted by the embodiments of the present disclosure have been disclosed directly or implicitly in the detailed description of the embodiments of the present disclosure. For example, various effects predicted according to embodiments of the present disclosure have been disclosed in the above-described detailed description.

Other aspects, advantages and prominent features of the present disclosure will become apparent to those skilled in the art from the above detailed description which disclosed various embodiments of the present disclosure taken in conjunction with the accompanying drawings.

Although embodiments of the present disclosure have been illustrated and described hereinabove, the present disclosure is not limited to the above-described specific embodiments, but may be variously modified by those skilled in the art to which the present disclosure pertains without departing from the gist of the present disclosure as disclosed in the accompanying claims. These modifications should also be understood to fall within the scope and spirit of the present disclosure.

## Claims

1. An analyte monitoring device comprising:
an analyte sensor of which at least a portion is located under a skin of a user and detects an analyte signal related to a glucose concentration;
a communication interface including at least one communication circuit;
a memory storing at least one instruction; and
at least one processor,
wherein the at least one processor executes the at least one instruction to:
activate the communication interface at a predetermined first time interval;
perform a authentication for a first signal for waking up the analyte monitoring device when the first signal is received from a user terminal device through the activated communication interface;
establish a communication channel between the analyte monitoring device and the user terminal device when the authentication is successful; and
transmit information related to the analyte signal to the user terminal device at a predetermined second time interval through the communication channel.

2. The analyte monitoring device of claim 1, wherein the first signal includes first identification information for establishing the communication channel, and
the at least one processor establishes the communication channel based on whether the first identification information matches second identification information pre-stored in the memory.

3. The analyte monitoring device of claim 1, wherein the at least one processor demodulates a first portion of the first signal to acquire a first value, the first portion modulated based on on-off keying (OOK), and
determines that the authentication is successful when the first value matches a second value pre-stored in the memory.

4. The analyte monitoring device of claim 1, wherein the at least one processor does not perform signal processing on the analyte signal while activating the communication interface at the predetermined first time interval, and
the signal processing includes at least one of a analog-to-digital conversion, a noise filtering, and a calibration.

5. The analyte monitoring device of claim 1, wherein, when a strength of the first signal is less than a preset value or the authentication fails, the at least one processor activates the communication interface at the predetermined first time interval without establishing the communication channel.

6. The analyte monitoring device of claim 5, wherein the at least one processor does not perform the authentication when the strength of the first signal is less than the preset value.

7. The analyte monitoring device of claim 1, wherein the first time interval is less than the second time interval.

8. The analyte monitoring device of claim 1, wherein the at least one processor transmits calibration information used to calibrate the analyte signal to the user terminal device through the communication channel.

9. A controlling method of an analyte monitoring device including an analyte sensor of which at least a portion is located under a skin of a user and detects an analyte signal related to a glucose concentration, the controlling method comprising:
activating a communication interface included in the analyte monitoring device at a predetermined first time interval;
performing a authentication for a first signal for waking up the analyte monitoring device when the first signal is received from a user terminal device through the activated communication interface;
establishing a communication channel between the analyte monitoring device and the user terminal device when the authentication is successful; and
transmitting information related to the analyte signal to the user terminal device at a predetermined second time interval through the communication channel.

10. The controlling method of claim 9, wherein the first signal includes first identification information for establishing the communication channel, and
the establishing of the communication channel is performed based on whether the first identification information matches second identification information pre-stored in the memory.

11. The controlling method of claim 9, wherein the performing of the authentication includes:
demodulating a first portion of the first signal to acquire a first value, the first portion modulated based on on-off keying (OOK); and
determining that the authentication is successful when the first value matches a second value pre-stored in the memory.

12. The controlling method of claim 9, wherein signal processing is not performed on the analyte signal while activating the communication interface at the predetermined first time interval, and
the signal processing on the analyte signal includes at least one of a analog-to-digital conversion, a noise filtering, and a calibration.

13. The controlling method of claim 9, further comprising, when a strength of the first signal is less than a preset value or the authentication fails, activating the communication interface at the predetermined first time interval without establishing the communication channel.

14. The controlling method of claim 13, wherein, when the strength of the first signal is less than the preset value, the authentication is not performed.

15. The controlling method of claim 9, wherein the first time interval is less than the second time interval.

16. The controlling method of claim 9, further comprising transmitting calibration information used to calibrate the analyte signal to the user terminal device through the communication channel.
